(19) **Europäisches Patentamt**

**European Patent Office**

**Office européen des brevets**

(11) **EP 1 588 711 A1**

(12) **EUROPEAN PATENT APPLICATION**

(43) Date of publication:
**26.10.2005 Bulletin 2005/43**

(51) Int Cl.⁷: **A61K 35/78**, A61K 45/06,
A61P 39/06, A61P 9/12

(21) Application number: **05252505.2**

(22) Date of filing: **21.04.2005**

(84) Designated Contracting States:
**AT BE BG CH CY CZ DE DK EE ES FI FR GB GR
HU IE IS IT LI LT LU MC NL PL PT RO SE SI SK TR**
Designated Extension States:
**AL BA HR LV MK YU**

(30) Priority: **21.04.2004 JP 2004125000**

(71) Applicant: **AOL Corporation
Suita-shi, Osaka-fu (JP)**

(72) Inventors:
• **Tabata, Akira
Suita-shi Osaka-fu (JP)**
• **Nakamura, Takumi
Kawachinagano-shi Osaka-fu (JP)**

(74) Representative: **Scott, Susan Margaret et al
Abel & Imray,
20 Red Lion Street
London WC1R 4PQ (GB)**

(54) **Composition trapping radicals in organism**

(57)     The object of the present invention is to provide a composition which is capable of going into erythrocyte or into cells, trapping not only oxygen radicals but also hydroxyl radicals, and preventing or improving various symptoms including hypertension, excessive sensitivity to cold, diabetes, Parkinson's disease, menstrual disorder, brain infarct, atopic dermatitis, hay fever, articular rheumatism, autonomic ataxia, and the like by oral intake.

The present invention relates to an invention of a composition trapping radicals in organism comprising the steps of:

roasting soy bean, wheat germ, adlay, husked rice germ, and rice bran, which are materials at a temperature of 50 to 150°C independently, followed by steaming them independently, and fermenting them independently with each of said materials by adding at least one species of edible microbes selected from microbes belonging to Aspergilius oryzae, and after fermentation, adding fine powders of at least green tea powders, sesame, rosemary, acerola, yuzu, carrot, Gymnema Sylvestre and green barley to each of said materials thereafter mixing, further followed by making the mixture ultra fine powders with 30 to 50μm.

**Description**

BACKGROUND OF THE INVENTION

Field of the Invention

The present invention relates to a composition trapping radicals in organism, and to be specific, the present invention relates to a composition capable of effectively trapping oxygen radicals and hydroxyl radicals in blood or cells, and in more detail, the present invention relates to a composition trapping radicals in organism capable of developing said effects by oral intake.

Description of the Background

Active oxygen or free radicals, with excessive presence in organism, cause inflammation, damage genes, cell membranes, or blood vessels, and generate lipid peroxide by oxidizing lipid in cell membranes or blood vessels. It is known that the excessive presence of active oxygen or free radicals causes various diseases including chronic inflammation such as atopic dermatitis, hepatitis, pneumonia, gastric inflammation, enteritis, asthma, gingival inflammation, or the like, adult diseases such as cancer, diabetes, cataracts, autonomic ataxia, hyperlipemia, hepatic cirrhosis, or the like, blood flow obstruction such as strokes, angina, coronary infarction, hardened arteries, hypertension, excessive sensitivity to cold, or the like, and other diseases such as Alzheimer's disease, Parkinson's disease, collagen disease, articular rheumatism, or the like.

Oxygen radical ($O_2\cdot$) is one representative of such active oxygen, and as enzyme which traps oxygen radical and removes it, superoxide dismutase (hereinafter "SOD") is exemplified. SOD removes oxygen radical which is present excessively in organisms based on the following reaction formula (chemical formula 1).

(chemical formula 1)

$$2\,O_2\cdot + 2\,H^+ \rightarrow H_2O_2 + O_2$$

Trapping oxygen radicals in organisms by a composition other than SOD by utilizing reactions shown in (chemical formula 1) is now studied by utilizing reactions represented in (chemical formula 1). A composition having a capability of trapping radicals by the above reaction represented in (chemical formula 1) is called an SODlike (function) composition. And above all, in order to overcome chronic disease, the method of easily continuing everyday is preferable, for example, by oral intake.

For above reason, several reports have been made regarding an SODlike composition which generates above reaction (chemical formula 1) in organisms by oral intake (for example, patent document 1).

(Patent document 1) Japan unexamined patent publication H6-284872

However, when the above reaction (chemical formula 1) is generated in blood, $H_2O_2$ generated with oxygen goes into erythrocyte, and under the presence of iron which composes said erythrocyte as catalyst, the following (chemical formula 2) Fenton reaction is generated. As a result, hydroxyl radical (OH·) is generated (reference is made to non-patent document).

(Chemical formula 2)

$$H_2O_2 + Fe^{2+} \rightarrow OH\cdot + OH^- + Fe^{3+}$$

(Non-patent document 1) T.Obata&Y.Yamanaka, Naunyn-Schmiedeberg's Arch Pharmacol(2002) 365:158-163

Like oxygen radical, this hydroxyl radical causes excessive oxidization of lipid in erythrocyte membrane or cell membrane and damages it. In order to prepare a composition for inhibiting such excessive oxidization of lipid, the composition should be capable of trapping hydroxyl radical in blood or cells effectively and of trapping oxygen radical as SODlike function as well.

Summary of the Invention

The object of the present invention is to provide a composition trapping radicals in organism capable of going into erythrocyte or inside of cells in blood or cells, having capability of trapping not only oxygen radical but also hydroxyradical, and of effectively inhibiting and improving several symptoms generated from active oxygen by oral intake.

As a result of intensive studies, the inventors of the present invention have solved the above problems by taking the following measures.

That is, the present invention relates to an invention of a composition trapping radicals in organism comprising the steps of:

roasting soy bean, wheat germ, adlay, husked rice germ, and rice bran, which are materials at a temperature of 50 to 150°C independently, followed by steaming them independently, and fermenting them independently by adding at least one species of edible microbes selected from microbes belonging to Aspergilius oryzae to each of said materials independently, and after fermentation, adding fine powders of at least green tea, sesame, rosemary, acerola, yuzu, carrot, Gymnema Silvestre and green barley to each of said materials, thereafter mixing, further followed by making the mixture ultra fine powders with 30 to 50μm.

Further, the compounding ratio of main ingredients of said composition trapping radicals in organism is preferably contained in 25 to 40 wt. % for soy bean, 25 to 35 wt. % for wheat germ, 3 to 10 wt. % for adlay, 3 to 20 wt. % for husked rice germ, 5 to 20 wt. % for rice bran, 15 to 20 wt. % for green tea powders, 1 to 10 wt. % for sesame powders, and 0.05 to 5 wt. % for other ingredients.

In addition, the present invention relates to an extract of a composition trapping radicals in organism which was extracted from said composition trapping radicals in organism.

Further, the present invention also relates to an invention of a composition trapping hydroxyl radicals within blood or within cells comprising the steps of:

roasting soy bean, wheat germ, adlay, husked rice germ, and rice bran, which are materials at a temperature of 50 to 150°C independently, followed by steaming them independently, and fermenting them independently by adding at least one species of edible microbes selected from microbes belonging to Aspergilius oryzae to each of said materials independently, and after fermentation, adding fine powders of at least green tea, sesame, rosemary, acerola, yuzu, carrot, Gymnema Silvestre and green barley to each of said materials thereafter mixing, further followed by making the mixture ultra fine powders.

Further, the present invention is the invention of a preparation method of a composition trapping radicals in organism having at least:

the first step of roasting soy bean, wheat germ, adlay, husked rice germ, and rice bran which are materials at a temperature of 50 to 150°C independently, followed by steaming them independently;

the second step of adding at least one species of edible microbe selected from microbes belonging to Aspergilius oryzae to each of said materials independently and fermenting them independently, and further, during this fermentation process, conducting cut back operation at least once in each fermentation material;

the third step of adding at least green tea powders, sesame powder, rosemary, acerola, yuzu, carrot, Gymnema Silvestre and green barley to each of said materials independently thereafter mixing; and

the fourth step of grinding mixture obtained in the third step into ultra fine powders of 30 to 50 μm.

Since the composition trapping radicals in organism of the present invention is capable of trapping not only oxygen radicals but also hydroxyradicals in blood and in cells by oral intake, the composition has a high inhibition effect against active oxygen. Further, the composition trapping radicals in organism of the present invention has the effect of inhibiting hemolysis caused by hydroxyradicals by going into erythrocyte in blood.

For this, reduction of erythrocyte caused by hydroxyradicals can be inhibited and transporting capacity of oxygen or nutrient in blood can be enhanced.

By the above function, the composition trapping radicals in organism of the present invention is excellent in inhibiting and improving effect of several symptoms caused by blood flow obstruction in particular, including stroke, hypertension, cardiac infarction, cancer, and the like. Among others, as shown in the animal testing and monitor test, the composition trapping radicals in organism of the present invention is highly effective in inhibiting hypertension and in improving excessive sensitivity to cold.

Further, since the composition trapping radicals in organism of the present invention is capable of protecting beta cells in pancreas islet tissue in a spleen which controls insulin secretion from radicals in addition to preventing blood flow obstruction as mentioned above, the composition is also effective in inhibiting and in improving diabetes. In this regard, it was proved that the composition trapping radicals in organism of the present invention has higher effect of inhibiting blood glucose level compared with the effect when catechin is used and it is assumed that the improving effect against diabetes is high.

In addition, in the composition trapping radicals in organism of the present invention, phytic acid is included, and said phytic acid is known to have improving effect of Parkinson' s disease. Therefore, when phytic acid is taken in in the form of the composition trapping radicals in organism of the present invention, since hydroxyradical trapping effect can be successfully developed at the same time, it is assumed that the improving effect of said Parkinson's disease will be enhanced.

Moreover, results of monitor tests mentioned later have shown that the composition trapping radicals in organism of the present invention is also effective not only in above symptoms but also inmenstrual disorder, diabetes, brain infarct, visceral disease (hepatitis C, gallbladder polyp, IgA nephropathy), constipation, atopic dermatitis, hay fever, arthrorheumatism, osteoporosis, disk herniation, gout, autonomic ataxia, hyperventilation syndrome, chronic headache, graves disease, cataracts, asthenopia, Morton's disease, and the like.

Moreover, the results of monitor tests have shown that the composition trapping radicals in organism of the present invention has improving effect of several symptoms not only in humans but also in animals like dogs and cats that are kept as pets.

Particularly, in preparing the composition trapping radicals in organisms of the present invention, it is noted that at least one species of edible microbes selected from microbes belonging to Aspergillius oryzae is added to each of said steamed materials independently after roasting so that said edible microbes infiltrate materials before fermentation process. Further, during the fermentation process, since cut back operation of materials is conducted at least once, condition for fermentation can be set even for materials which are hard to ferment depending on the liability of fermentation. By undergoing this process, such composition can be prepared in which all the materials can have low molecular weight. Thus, by making all the materials have low molecular weight, absorption by oral intake gets easier and effective intake of active ingredient can be realized.

In addition, since the temperature of roasting or steaming materials is set to be 50 to 150 °C, effective fermentation can be realized during the fermentation process after roasting and steaming process and at the same time, active ingredients are kept undestroyed by heat during the said roasting or steaming process.

Further, the composition trapping radicals in organisms of the present invention is an ultra fine powder with a diameter of 30 to 50 µm. Normal powder has the diameter of about 300µm even when the powder is ground to a fine powder. However, the powder of the present invention is an ultra fine powder with a diameter of 30 to 50 µm, it is excellent in digestion and absorption, and active ingredients can be taken effectively in a body.

In addition, since the composition trapping radicals in organisms of the present invention has the same effect as it is taken in as powdery composition when it is made in the form of essence, using said essence has enabled the body intake easy as other ways than oral intake.

In addition, it is assumed that the composition trapping radicals in organisms of the present invention is effective in trapping not only above oxygen radicals or hydroxyl radicals but also active oxygen materials such as singlet oxygen or hydrogen peroxide solution.

Brief description of the drawings

Fig.1 is an explanatory diagram of a measuring method of a diameter of hemolysis in erythrocyte.

Fig.2 is a graph showing changes of blood glucose level of mice with time in the experiment for confirming improving effects for diabetes.

Fig.3 is a graph showing changes of blood glucose level of mice with time in the experiment for confirming improving effects for diabetes regarding methanol extract (SMeEx) of the composition trapping radicals in organisms of the present invention.

Fig.4 is a graph showing the influence of alloxane on lipid peroxide levels of erythrocyte and the influence of (+) -catechin and S.AOL MeOH extract on the effect.

Fig.5 is a graph showing the influence on the change in body weight.

Detailed description of the preferred embodiments

Explanation goes further regarding the composition trapping radicals in organism of the present invention. The composition trapping radicals in organisms of the present invention refers to a composition capable of removing said radicals in organisms by trapping radicals such as oxygen radicals or hydroxyl radicals inside of organisms, particularly in blood or cells and reacting. Inside of organisms refers to inside of the body of living matters.

(Preparation method)

The first process for preparing the composition trapping radicals in organism of the present invention is the process of roasting each material of soy bean, wheat germ, adlay, husked rice germ, and rice bran followed by steaming. However, in addition to the above materials, secondary materials may be added.

"Roasting" is defined to roast and toast materials by infra-red ray and, in particular, by far infra-red ray. "Steaming" means heating materials by vapor. Each material can be heated which is required for making materials low molecular weight by undergoing steaming process after roasting. In the process of steaming and roasting, most preferable temperature for effectively making materials low molecular weight without getting materials burnt ranges from 50 to 150°C. Since most preferable condition for temperature in the process of steaming and roasting materials varies depending on each character and amount of materials like soy bean, wheat embryos, adlay, husked rice germ, and rice bran, suitable temperature can be determined among said range of 50 to 150°C. The same applies to the time for steaming and roasting materials. In preparing the composition trapping radicals in organism of the present invention, it is noted that great amount of phytic acid is included therein since as mentioned above, the composition mainly consists of soybean, wheat germ, adlay, husked rice germ, and rice bran as materials. To be specific, analytical result shows that 593 mg of phytic acid is included per 100 g of the composition trapping radicals in organisms of the present invention. The content of phytic acid is far greater than that of chlorella (342mg of phytic acid per 100 g thereof),and green juice (285mg of phytic acid per 100 g thereof). Phytic acid has the function as an inhibitor capable of preventing generation of hydroxyl radicals since phytic acid inhibits Fenton reaction as mentioned above (chemical formula 2) by chelating with iron.

The second process is a process of fermenting materials by adding at least one species of edible microbes selected from microbes belonging to Aspergillus oryzae to each of materials undergoing said first process. "Fermentation" shouldbe broadly interpreted and refers to the process in which microbes such as yeast or bacteria decompose organic compounds. Here, Aspergillius oryzae added for fermentation means a kind of edible microbe. At least one species of edible microbes used for fermentation process in the present invention is an edible microbe selected from said Aspergillus oryzae. By undergoing fermentation process using edible microbe belonging to Aspergillius oryzae, the composition can have low molecular weight and further, SODlike function can be improved. Regarding addition of edible microbes, it is preferable to combine said edible microbes with materials well thereby making said edible microbes evenly before fermenting composition.

Further, during fermentation process, a so called "cut back" operation is applied at least once to each of the materials. In said process, an operation of flaking materials under fermentation is conducted. This cut back operation is applied at least once every day and it is preferable that this operation is carried out by hand. By carrying out the cut back operation by hand every day, unfermented portion in the materials can be discovered and said unfermented portion can be targeted for fermentation. With such an operation, even for materials which are hard to ferment, fermentation becomes easier and low molecular weighted composition trapping radicals in organisms of the present invention as a whole can further be promoted.

The third process is a process of adding green tea powders, sesame powder, rosemary, acerola, yuzu, carrot, Gymnema Sylvestre and green barley to each of fermented materials obtained by said second process, followed by mixing, thereby obtaining a composition trapping radicals in organisms.

Green tea powders are added with a view to containing catechin, vitamin C or derivative thereof. Here, green tea powders are added after fermentation in order to inhibit decomposition of active ingredients such as vitamin C which are included in green tea powders.

In addition, sesame powder is added with a view to containing antioxidants such as tocopherol, sesamine, and sesamenol, which are included in sesame powder. Here, sesame powder is added after the completion of fermentation process since sesame is hard for fermentation due to large containment of oils and it becomes obstacle for fermentation of main ingredients. Black sesame as well as white sesame can be employed as sesame.

Further, as antioxidants, rosemary, acerola, yuzu, carrot, Gymnema Sylvestre, and green barley are added. By containing several species of antioxidants such as rosemary, acerola, yuzu, and carrot, more effective antioxidant function can be developed compared with when single antioxidant is included.

Further, Gymnema Sylvestre is included for the purpose of inhibiting absorption of sugar content within the intestine and inhibiting caloric intake. Green barley is included for the purpose of taking in vitamins and minerals in good balance.

The fourth process is a process of grinding. In the fourth process, mixture obtained in the third process is further ground and said mixture is further made into ultra fine powders with a diameter of 30 to 50μm. The size of powders referred here is an average size of each powder. For grinding said mixture, known grinding machines can be used and to be specific, jet mills and turbo mills can be exemplified.

(Compounding ratio)

Preferable compounding ratio of main ingredients of the composition trapping radicals in organisms in the present invention is 25 to 40 wt % for soy bean, 25 to 35 wt % for wheat germ, 3 to 10 wt % for adlay, 3 to 20 wt % for husked rice germ, 5 to 20 wt% for rice bran, 15 to 20 wt % for green tea powders, 1 to 10 wt % for sesame powder, and 0.05 to 5wt % for other ingredients. This is the compounding ratio of the composition trapping radicals in organisms of the present invention which was used in the experiments for confirming effects as mentioned below and this is the compounding ratio proved to be effective by the experiments. From the experiments as shown below, it is assumed that being within the range of said compounding ratio enables the trapping of not only oxygen radicals but also hydroxyl radicals in blood or within cells and has shown synergistic effect by effectively trapping both radicals.

(Shapes of composition, extract)

Although the shape of the composition trapping radicals in organisms obtained by undergoing said first to third process is powdery, the shape can be a solid form or pellet form to make the composition a tablet form by ramming down powders.
Further, the composition trapping radicals in organisms of the present invention can be utilized as extracts of the composition trapping radicals in organisms by extracting its essence. Although one example of extracting method of essence is shown below, the extracting method is not limited to this.
The composition trapping radicals in organisms of the present invention is dissolved into methanol solution (methanol: water = 4:1, in terms of volume) and is filtered. The filtrate is concentrated by evaporation. Since methanol can be removed by evaporation, essence in a liquid form is generated in this stage. Further, by removing liquid part from this essence in a liquid form by a vacuum pump, essence in a solid form can be obtained as a residue. And by encapsulating this, it can be used as a soft capsule. Further, as ways for intake other than oral intake, said liquid form essence can be applied as liniments or can be utilized by directly applying liniments obtained by kneading said powdery composition with liquid to the skin.

(Method of intake)

Amount of dosage of the composition trapping radicals in organisms for preventing and improving several symptoms caused by said excessive amount of radicals in organisms varies depending on kinds of symptoms and seriousness of symptoms. To cite one example, for improving excessive sensitivity to cold, referring to experiments for confirming effects as below, when 60mg or greater amount of composition is taken per body weight (kg) per day, in many cases effects are brought about in one month. In other words, in the case of a person with a body weight of 50 kg, the desirable amount of intake is about 3g per day. However, even when the amount of intake is less than said desirable amount, depending on individual difference and symptom difference, the same effect can be brought about and on the other hand, the more amount one takes in, the more effect one can expect. For information, since the composition of the present invention is found to consist of materials which can be used as foods, even when one takes in large amount, there are no side effects as with general foods.

Example

(Experiments for confirming effects by using erythrocyte; Example 1)

In order to confirm the effects of the composition trapping radicals in organisms of the present invention going into erythrocyte or within cells in blood, following experiments by a red cell method stated in the non patent document 2 was conducted. The details are as follows.
(Non patent document 2) Yutaka Matsuoka, Kanako Wakayama, Ayano Takashina and Yoshie Yamada, "Simple screening method for bioactive substances protecting nerves from active oxygen related injury". Japan. J. Pharmacol., 88(Supl. 1), 141, (2002).

(Principle)

Agar mediummixed with blood is prepared and when glucose oxydase (hereinafter it is called "GOD") is added to a paper disc placed on said medium, glucose in the medium and oxygen are catalyzed by GOD, and $H_2O_2$ is generated. While GOD cannot pass through cell membrane, generated $H_2O_2$ goes into erythrocyte or within cells and Fenton reaction is caused by iron as a catalyst in blood cells. By this, hydroxyradical (OH·) is generated and hemolysis occurs by the lipid peroxidation of an erythrocyte membrane. Condition of hemolysis is observed as circle shaped hemosysis. When the disc with a test material added which is capable of going into erythrocyte or within cells and trapping hydroxylradicals is placed next to the disc with GOD added, hemolysis is inhibited and as a result, the radius of circle shaped hemosysis becomes smaller. Since the more powerful the capability of trapping hydroxyl radicals is, the smaller the radius of circle shaped hemosysis becomes, by measuring said radius, the power of capability of trapping hydroxyl radicals by a test material can be measured.

(Experiment method)

| (Preparation of blood agar medium) | |
|---|---|
| NaCl | 6.90 g |
| KCl | 0.35 g |
| Glucose | 20.0 g |
| Na2HPO4 · 12H2O | 2.90 g |
| Na2HPO4 · 2H2O | 0.296 g |

20 g of L glucose: Krebs-10mMphosphate-buffer (pH7.4) comprising above ingredients is dissolved into purified water to make whole amount 1 L, followed by adding 15 g of agar and sterilizing for 20 minutes with autoclave, thereafter dispensing into test tubes with 6 mL each, thereby preparing phosphoric acid buffer agar medium.
Next, blood was collected from a heart of a mouse adding 3.8 % % sodium citrate in not less than 10% with respect to the total amount of total collected blood.
Physiological saline including 0.5 % glucose was added thereto and cleaned three times by centrifugation (1600rpm, 10 minutes). Next, taken blood was diluted into almost double the amount by physiological saline including 0.5 % glucose and allowed the fluid to drip into a sterilized petri dish. Then 6 ml of said phosphoric acid buffer agar medium with a temperature of 60 °C after dissolution by an autoclave was poured, followed by mixing evenly and solidifying, thereby preparing blood agar medium.

(Preparation of test articles)

25 g of the composition trapping radicals in organism of the present invention was extracted in 200 mL(methanol 160mL and water 40 mL) and was filtered, followed by concentrating by an evaporator and drying with a vacuum pump. As an essence, 4.31 g of residual was obtained. This residual was dissolved in physiological saline and after applying ultra sound wave, the residual was melt by heating with boiling water. Although residue remains in the case of 300 mg/mL and of 1000 mg/mL, supernatant liquid was used.
The experiment by this essence is called Example 1.

(Test method)

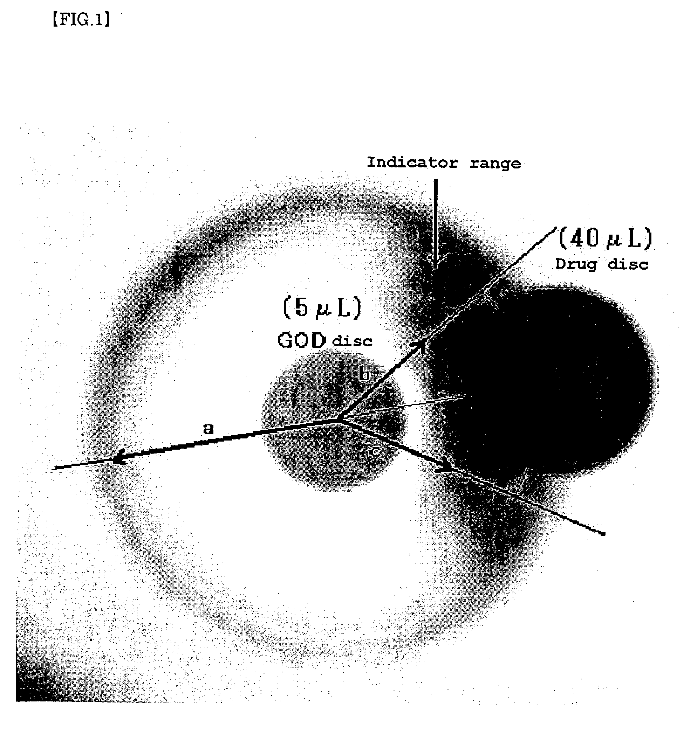

100 mg/mL of the essence of the above invention which is a test material was dissolved in physiological saline, thereby preparing the test solution of Example 1. In addition, GOD was dissolved in physiological saline, thereby preparing GOD solution(60,200,600U/mL). Next, on said blood agar medium, paper discs whose thickness is 6 mm (thin disc) and 8mm (thick disc) were placed keeping a space of 2mm therebetween. 5μL of GOD solution was added to a paper disc with a thickness of 6 mm and 40 μL of test solution was added to a paper disc with a thickness of 8 mm. Then, argon gas which is inert gas was filled in the petri dish and was cultured for a night at a temperature of 37°C, thereafter measuring a radius of circle shaped hemolysis. A method of measurement is shown in Fig.1. A radius 'a' of circle shaped hemolysis shown in Fig. 1 was called 'control' and the average of 'b' and 'c' was called 'test' , thereby measuring the raius. Considering the effect of drugs, measurement was made 1 mm outside of tangent line. Measurement value was set by subtracting 3mm of the disc radius from this measurement value and was used. As explained in the above principle, by said measurement method, it is assumed that the smaller value of test compared with the value of control shows greater capability of trapping hydroxyl radicals in blood.

(Result)

A radius of circle shaped hemolysis in GOD solution 60, 200, 600 U/mL is shown in Table 1.

Table 1

| Radius of circle shaped hemolysis (mm) by using erythrocyte | | | |
|---|---|---|---|
| Example 1 | Concentration of G O D (U/mL) | | |
| | 60 | 200 | 600 |
| test | 1.02 | 1.17 | 1.25 |
| control | 5.95 | 6.57 | 8.00 |

From the above result, it is recognized that the composition trapping radicals in organism in the present invention is capable of going into erythrocyte or cells and has capability of trapping hydroxyl radicals. From this, since the composition trapping radicals in organisms of the present invention is capable of trapping and removing hydroxyl radicals as well as oxygen radicals in blood, it is assumed that more effective prevention and improvement for blood flow obstruction is realized.

(Experiments for confirming effects using laboratory rats)

Experiments for confirming effects for improving excessive sensitivity to cold and effects for inhibiting blood pressure elevation were conducted using laboratory rats. The detail is as follows.

(Experiments for confirming effects for improving excessive sensitivity to cold; Example 2, Comparative Example 2)

(Condition for administration)

In order to examine the effects of a composition trapping radicals in organisms of the present invention for improving excessive sensitivity to cold, animal testing using rats was conducted. 10 rats provided with a composition trapping radicals in organisms of the present invention (Example 2) and another 10 rats as comparison (Comparative Example 2) were used.

As a method of administration, a composition trapping radicals in organisms of the present invention was dissolved in methyl cellulose (hereinafter called as "medium material"), thereby making the 5wt% solution (hereinafter called as "test article") and said solution was administered to rats.

Further, the composition was administered to rats so that the total amount of administration of a composition trapping radicals in organisms of the present invention is 2000mg/kg per weight of rats in 29 days. As shown below, since the composition was administered to rats 57 times in total, the amount of active ingredients per weight of rats is about 35 mg/kg per once.

Although the amount of administration of test articles differs depending on weight of each rat, if the weight of rats is about 200g, the amount of administration of test materials is about 0.14 mL per once. On the other hand, the same amount (about 0.14 mL) of medium materials was administered to rats for Comparative Examples in the same times by oral intake. For information, the rats took in water and solid feed freely during the experiment. As solid feed, "CRF-1" manufactured by Oriental Yeast Co., Ltd. was used. During the experiment, weight of each rat was measured twice a week.

(A method of for confirming effects for excessive sensitivity to cold)

Rats were kept under fast state for 24 hours and left at rest for more than 30 minutes after applying the above adjustment for 28 days. The temperature of a body surface was measured using a medical thermographic apparatus ("Thermo viewer JTG manufactured by JEOL Co., Ltd.), which was the body temperature before water immersion. After measuring the body temperature before experiment, test materials (about 0.14 mL) were administered to the rats for Example 2, and medium materials (about 0.14 mL) were administered to the rats for Comparative Example 2, respectively. In 30 minutes after administration, the rats were immersed in water of a temperature of 15°C from the neck down for 15 minutes. When water immersion was over, the water on body surface was wiped out immediately and temperature of a body surface was measured using said thermographic apparatus 5, 10, 20, 30, 40, 50, 60, 80, 100, and 120 minutes after water immersion.

For information, temperature of a body surface was measured by calculating the regional area by image having the temperature of 24°C or more in the body surface of rats photographed by said thermographic apparatus and pixel which represents the number of pixel is used as a unit. That is, the large number of pixel shows the existence of region having high temperature of a body surface (region having a temperature of 24 °C or more) in the wide range.

(Result)

(Change in body weight)

Table 2 (a) shows the average value of body weight of each rat during the period of administration for 28 days. For information, underneath is the error range shown (the same applies to Table 2 (b)). No difference in the average body weight was observed between rats for Example 2 and rats for Comparative Example 2.

**Table 2 (a) Test for confirming effects of improving excessive sensitivity to cold: Average value of body weight (g)**

| | Time passage (days) | | | | | | | | |
|---|---|---|---|---|---|---|---|---|---|
| | 1 | 4 | 8 | 1 1 | 1 5 | 1 8 | 2 2 | 2 5 | 2 8 |
| Example 2 (n=10) | 202 ±2 | 209 ±3 | 218 ±3 | 224 ±3 | 231 ±3 | 235 ±3 | 245 ±2 | 248 ±3 | 234 ±3 |
| Comparative Example 2 (n=10) | 202 ±2 | 211 ±3 | 221 ±3 | 224 ±3 | 233 ±3 | 235 ±3 | 243 ±3 | 247 ±2 | 232 ±3 |

(Change in temperature of body surface)

Table 2 (b) shows the average value of each rat for Example 2 and for Comparative Example 2 in each time in the experiment confirming the effect for excessive sensitivity to cold. In addition, the indications marked upper right of the values in the Example are the indications showing statistical significance, "*" shows that the risk rate is less than 5 % ($P<0.05$), and "**" shows that the risk rate is less than 1 % ($P<0.01$).

**Table 2 (b) Test for confirming effects of improving excessive sensitivity to cold: Average value of regional area where the temperature of body surface is not less than 24°C ($\times 10^2$ Pixel)**

| | Time passage (minutes) | | | | | | |
|---|---|---|---|---|---|---|---|
| | Before water | 0 | 5 | 10 | 20 | 30 | 40 |
| | immersion | | | | | | |
| Example 2 (n=10) | 242 ±8.4 | 149 ±15.1 | 189 ±11.3 | 189 ±9.2 | 214 ±11.7 | 243* ±6.0 | 267* ±8.2 |
| Comparative Example 2 (n=10) | 211 ±8.4 | 117 ±15.1 | 148 ±11.3 | 172 ±9.2 | 207 ±11.7 | 224 ±6.0 | 233 ±8.2 |

| | Time passage (minutes) | | | | |
|---|---|---|---|---|---|
| | 50 | 60 | 80 | 100 | 120 |
| Example 2 (n=10) | 267** ±8.3 | 241* ±4.6 | 244 ±5.2 | 253* ±6.0 | 237* ±4.7 |
| Comp. Example 2 (n=10) | 231 ±8.3 | 222 ±4.6 | 233 ±5.2 | 237 ±6.0 | 218 ±4.7 |

It is found that compared with rats for Comparative Example 2, rats for Example 2 maintained higher temperature of a body surface in wider range of a body surface before water immersion.

Therefore, the rats administered test materials were found to be capable of maintaining higher temperature of a body surface compared with rats for Comparative Example in any point of time after water immersion. Thus, it is assumed that the rats administered test materials did not decrease body temperature tremendously even they were cooled by water since they came to acquire body habit of maintaining high body temperature in wide range of body surface as usual state, while the rats for Comparative Example did not.

(Confirming effects for improving hypertension; Example 3, Comparative Example 3)

In order to examine effects of the composition trapping radicals in organisms of the present invention for improving hypertension, animal testing was conducted using spontaneously hypertensive rats which elevate blood pressure with advancing age (hereinafter called "SHR"). 10 SHRs administered the composition trapping radicals in organisms of the present invention (Example 3) and another 10 SHRs as comparison (Comparative Example 3) were used.

(Condition for administration)

As a method of administration, as in Example 2, a composition trapping radicals in organisms of the present invention was dissolved into metyl cellulose, thereby making 5wt % solution ("medium material") and said solution was administered to SHRs. Further, the composition was administered to rats so that the total amount of administration of a composition trapping radicals in organisms of the present invention is 2000mg/kg per per weight of SHRs in 36 days. As shown below, since the composition was administered to SHRs 72 times in total, the amount of active ingredients per weight of SHRs is about 28 mg/kg per once. If the weight of rats is about 200g, the amount of administration of test materials is about 0.11 mL per once. On the other hand, the same amount (about 0.11 mL) ofmediummaterials were administered to SHRs for Comparative Examples in the same times by oral intake. For information, the rats took in water and solid feed freely during the experiment. As solid feed, "CRF-1" manufactured by Oriental Yeast Co., Ltd. was used as in Example 2. During the experiment, weight of each SHR, systolic blood pressure, average blood pressure, and diastolic blood pressure thereof were measured once a week. After administration for 36 days, a three-day period during which administration was stopped (hereinafter called "drug holidays") was provided, and then above measurement was conducted.

(Method for confirming blood pressure)

Systolic blood pressure, average blood pressure, diastolic blood pressure, and heart rate were measured using "BP-98A" (manufactured by Softron Co., Ltd.) which is the bloodless automatic blood pressure measuring apparatus under non-anesthesia by TAIL CAFF method. For information, before measuring blood pressure and heart rate, in order to elevate the body temperature of SHRs, SHRs were put into the warmth-retention box with a temperature of 38°C.

(Result)

(Change in body weight)

Table 3 (a) shows the average value of body weight of each SHR. For information, underneath is the error range shown (the same applies to Tables 3 (b) to (e)). No difference in the average body weight was observed between SHRs for Example 3 and rats for Comparative Example 3.

Table 3 (a) Test for confirming effects of improving hypertension: Average value of body weight (g)

| | Time passage (days) | | | | | | |
|---|---|---|---|---|---|---|---|
| | 1 | 8 | 15 | 22 | 29 | 38 | 3 days after drug holidays |
| Example 3 (n=10) | 138 ±2.0 | 178 ±2.1 | 218 ±3.7 | 239 ±3.7 | 258 ±4.3 | 272 ±4.5 | 276 ±4.2 |
| Comparative Example 3 (n=10) | 138 ±1.8 | 178 ±2.3 | 220 ±3.3 | 239 ±3.4 | 259 ±3.6 | 273 ±3.9 | 278 ±4.2 |

(Change in blood pressure)

Table 3 (b) shows the average value of systolic blood pressure of each SHR in Example 3 and in Comparative Example 3 in each time.

Table 3 (c) shows the average value of average blood pressure.

Table 3 (d) shows the average value of diastolic blood pressure.

Table 3 (e) shows the average value of heart rate. In addition, the indications marked upper right of the values in the Example are the indications showing statistical significance , "*" shows that the risk rate is less than 5 % (P<0.05), and "**" shows that the risk rate is less than 1 % (P<0.01).

Table 3 (b) Test for confirming effects of improving hypertension: Average value of systolic blood pressure (mmHg)

| | Time passage (days) | | | | | | | |
|---|---|---|---|---|---|---|---|---|
| | 0 | 1 | 8 | 15 | 22 | 29 | 38 | 3 days after drug holidays |
| Example 3 (n=10) | 113 ±2.1 | 120 ±3.2 | 143 ±3.2 | 166 ±2.1 | 177** ±3.4 | 193** ±1.9 | 197** ±2.1 | 202 ±3.3 |
| Comparative Example 3 (n=10) | 113 ±2.2 | 121 ±2.8 | 154 ±4.2 | 167 ±3.3 | 191 ±3.6 | 205 ±2.6 | 213 ±1.4 | 208 ±2.0 |

Table 3 (c) Test for confirming effects of improving hypertension: Average value of average blood pressure (mmHg)

| | Time passage (days) | | | | | | | |
|---|---|---|---|---|---|---|---|---|
| | 0 | 1 | 8 | 15 | 22 | 29 | 38 | 3 days after drug holidays |
| Example 3 (n=10) | 88 ±2.5 | 96 ±2.5 | 118 ±4.2 | 138 ±1.9 | 148* ±3.9 | 165* ±4.3 | 171** ±2.8 | 174 ±4.0 |
| Comparative Example 3 (n=10) | 87 ±3.0 | 96 ±3.0 | 124 ±4.0 | 139 ±3.6 | 162 ±3.6 | 178 ±2.5 | 187 ±2.4 | 180 ±1.6 |

Table 3 (d) Test for confirming effects of improving
hypertension: Average value of diastolic blood pressure(mmHg)

| | Time passage (days) | | | | | | | |
|---|---|---|---|---|---|---|---|---|
| | 0 | 1 | 8 | 1 5 | 2 2 | 2 9 | 3 8 | 3 days after drug holidays |
| Example 3 (n=10) | 75 ±2.8 | 84 ±2.1 | 105 ±4.4 | 125 ±1.9 | 133* ±4.6 | 151 ±5.9 | 159** ±3.5 | 161 ±4.5 |
| Comparative Example 3 (n=10) | 74 ±3.5 | 83 ±3.4 | 110 ±4.3 | 124 ±4.0 | 148 ±3.7 | 164 ±2.7 | 175 ±3.0 | 167 ±1.8 |

Table 3 (e) Test for confirming effects of improving

17

hypertension: Average value of heart rate(beat/min.)

| | Time passage (days) | | | | | | | |
|---|---|---|---|---|---|---|---|---|
| | 0 | 1 | 8 | 1 5 | 2 2 | 2 9 | 3 8 | 3 days after drug holidays |
| Example 3 (n=10) | 414 ±5.5 | 427 ±8.7 | 437 ±11.0 | 424 ±10.9 | 430 ±11.7 | 425 ±7.9 | 452 ±15.2 | 428 13.7 |
| Comparative Example 3 (n=10) | 422 ±7.9 | 436 ±10.5 | 423 ±8.8 | 417 ±11.1 | 424 ±5.4 | 424 ±7.7 | 459 ±7.0 | 422 ±7.6 |

FromTable 3 (e), no significant difference was observed between the heart rate of Example 3 and that of Comparative Example 3.

On the other hand, from Tables 3 (b), (c), and (d), in Example 3, regarding blood pressure (systolic blood pressure, average blood pressure, and diastolic blood pressure) on and after 22 days from the day of administration, more significantly low value was observer compared with Comparative Example 3. Further, no get back elevation of blood pressure was observed in SHRs for Example 3 after three-day drug holidays. From this, it was found that in SHRs administered the composition trapping radicals in organisms of the present invention, inhibition effect for blood pressure elevation was observed.

(Test for confirming effects of improving diabetes: Examples 4-1,4-2,Comparative Examples 4-1,5-1)

In order to test the effects of improving diabetes by the composition trapping radicals in organism of the present invention, animal experiments on alloxane administeredmice were conducted.

Alloxane administered mice develop diabetes due to generation of active oxygen within the body at the time of alloxane metabolism.

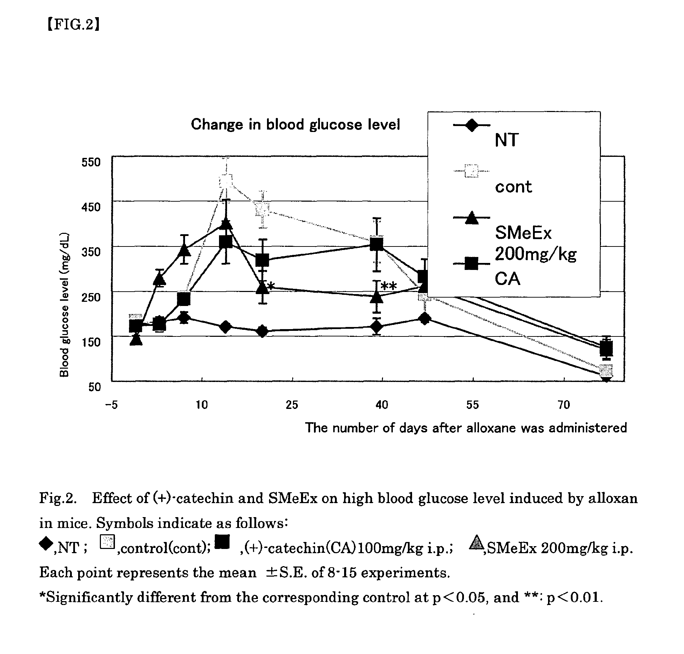

In other words, variation per day was tracked regarding blood glucose level of alloxane diabetes mice administered alloxane by 200mg/Kg with respect to the body weight thereof. Fig.2 shows the result. As Example 4-1, the composition trapping radicals in organism were administered to mice by 200mg/kg with respect to the body weight thereof (SMeEx of Fig.2).

As comparison, mice with only alloxane administered (Comparative Example 4-1 represented as control in Fig.2) and mice with catechin (+) administered by 100mg/kg with respect to the body weight thereof (Comparative Example 5-1 represented as CA in Fig.2) were used. In addition, in Fig.2, mice with nothing administered including alloxane were also shown represented as NT. Further, each point in Fig. 2 shows the average value of ±S.E in the number of experimental samples in 8 to 15.

As a result, as shown in Fig.2 (influence on blood glucose level), along with the administration of alloxane two times, on 0th day and on 7th day, increase in blood glucose level in the control group, the catechin group (CA group), and the group of composition trapping radicals in organism of the present invention (the SMeEx group)was observed. In the CA group, on 20th day, compared with the control group, decrease in blood glucose level was observed. However, when blood glucose level was measured on 39th day, compared with the level on 20th day, blood glucose level increased to a certain degree and no difference was observed between the CA group and the control group. In the SMeEx group, on 3rd day and 7th day, compared with the control group, blood glucose level increased compared with the control group, however, in the measurement on 14th day, 20th day, and 39th day, decrease in blood glucose level was observed. In particular, after 20th day and after 39th day, statistically significant value of $P<0.01$ and $P<0.05$ respectively shows that blood glucose level decreased.

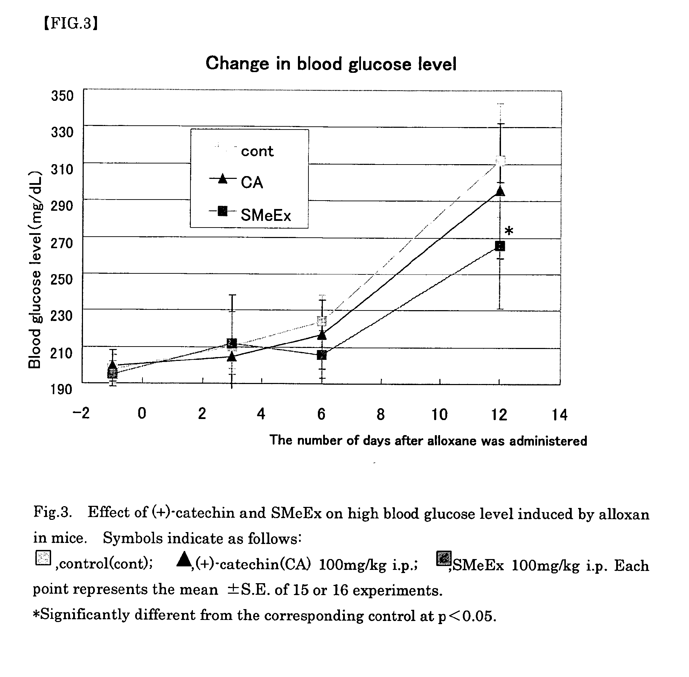

In addition, evaluation was made on the group of 100 mg/kg i.p. administration of methanol extract of the composition of trapping radicals in organism in the present invention (SMeEx) as shown as Example 4-2. The result is shown in Fig.3. From Fig. 3, methanol extract (SMeEx) was observed to be statistically significant in the decrease of blood glucose level ($P<0.05$).

For information, the effect of the decrease in blood glucose level was stronger in the methanol extract administration group (SMeEx) than in the CA group. In Fig.3, SMeEx is the methanol extract of the composition trapping radicals in organism of the present invention. CA is the catechin group, cont shows the control group, and CA and cont put down with the same evaluation result as in Fig. 2. Further, each point in Fig. 3 shows the average value of $\pm$S.E in the number of experimental samples in 15 or 16.

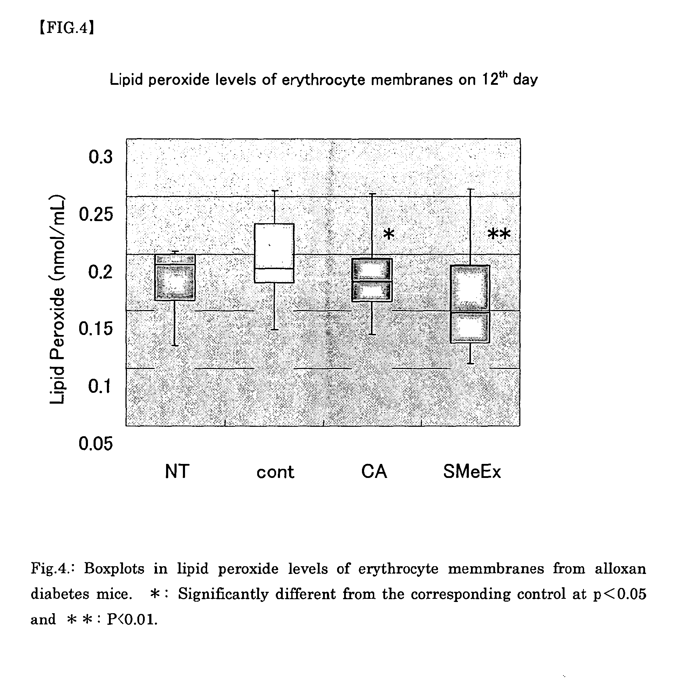

Next, regarding samples of 12th day after the administration of alloxane shown in Fig. 3, the effect on lipid peroxide level (n mol/mL) of erythrocyte membranes was evaluated. The result is shown in Fig.4. According to the figure, compared with the non-treated group (NT), the level of lipid peroxide tended to increase in the control group (cont) in which alloxane was administered. Regarding this tendency of increase in lipid peroxide due to alloxane, like the influence on blood glucose level, statistically significant decrease in lipid peroxide level of erythrocyte membranes was observed in the catechin group (CA) and in methanol extract group (SMeEx) of Example 4-2. Regarding the examination on significant difference, ranking of examination of Wilcoxon was employed. As a result, respectively, significant difference was observed between the control group and the CA group of $P<0.05$ and between the control group and the methanol extract group of the composition of trapping radicals in organisms of the present invention (SMeEx) of $P<0.01$. As seen in the result of blood glucose level, regarding the effect on the decrease in lipid peroxide, the tendency was stronger in the SMeEx group than in the CA group.

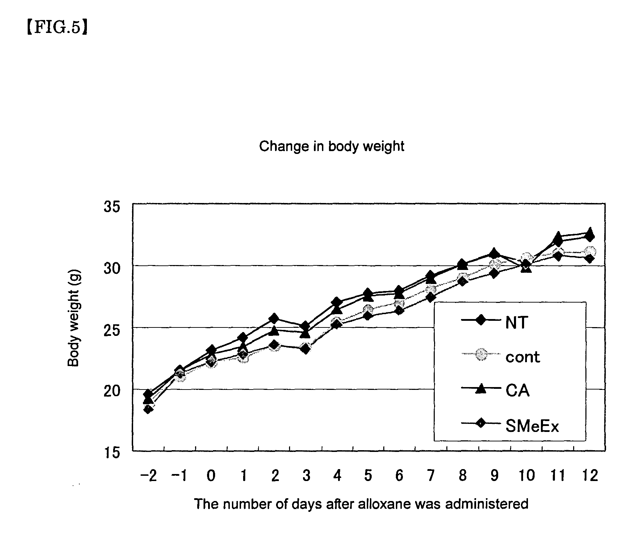

Regarding the effect on the change in the body weight (Fig.5), no difference was observed in any groups. That is, by the drug administration, influence such as toxic effect was not observed. For information, Fig.5 shows the influence of the non-treated group (NT), the control group (cont), the catechin group (CA), and methanol extract group of Example 4-2 (SMeEx) on the body weight corresponding to Figs. 3 and 4.

(Experiment for confirming effects by subjects)

In order to confirm the improving effect of the composition trapping radicals in organisms of the present invention for several symptoms of human body, we asked people suffering from several symptoms to be subjects for monitoring the effect of the composition trapping radicals in organisms of the present invention by way of oral intake. For information, in asking people for becoming subjects, 3 g of bagged powdery composition trapping radicals in organisms of the present invention was provided.

(Examples 5 to 12; improving case of hypertension)

(Example 5)

Male, 58 years old. He has been suffering from hypertension and the systolic blood pressure (so called the highest blood pressure, which we call the highest blood pressure hereinafter) was 180 and diastolic blood pressure (so called the lowest blood pressure, which we call the lowest blood pressure hereinafter) was 90. When he took one bagful of the composition trapping radicals in organism of the present invention every evening, in three months, blood pressure went down to a normal level with the highest blood pressure of 125 and with the lowest blood pressure of 80.

(Example 6)

Male, 53 years old. He has been suffering from hypertension and he took an antihypertensive drug. However, due to allergy of drugs, he had a rash all over his body. When he took one bagful of the composition trapping radicals in organism of the present invention everyday, in two months, blood pressure went down to a normal level and in three months, most rash has disappeared.

(Example 7)

Male, 57 years old. He has been suffering from hypertension with blood pressure of 160 to 170 as the highest and of 100 to 110 as the lowest. When he took two to three bagfuls of the composition trapping radicals in organism of the present invention every morning, in six months, blood pressure went down to 150 to 160 as the highest and 90 to 100 as the lowest.

(Example 8)

Male, 58 years old. Although he took an antihypertensive drug for 25 years, his blood pressure was still high of 135 as the highest and of 90 as the lowest. When he took the composition trapping radicals in organism of the present invention about four times a day, in one month, blood pressure went down to 130 as the highest and 76 as the lowest.

(Example 9)

Female, 66 years old. Although she took an antihypertensive drug every day for two years, her blood pressure was still high of 140 as the highest and of 100 as the lowest. When she took the composition trapping radicals in organism of the present invention about twice a day, in two months, blood pressure went down to 136 as the highest and 84 as the lowest.

(Example 10)

Male, 70 years old. Although he kept taking an antihypertensive drug, his blood pressure was still high of 170 to 190 as the highest and of 96 to 102 as the lowest. When he took two to three bagfuls of the composition trapping radicals in organism of the present invention a day, in two weeks, he has felt better. When he measured blood pressure, it went down to 126 as the highest.

(Example 11)

Male, 65 years old. He has been suffering from hypertension and his blood pressure was 180 as the highest and was 120 as the lowest. He used to take an antihypertensive drug. When he kept taking three bagfuls of the composition trapping radicals in organism of the present invention a day, blood pressure has become stabilized of 140 as the highest and of around 95 as the lowest even without taking said antihypertensive drug for about a week. Further, he has no headache any longer and can sleep soundly.

(Example 12)

Male, 41 years old. He was diagnosed as hypertension with blood pressure of 210 as the highest and of 130 as the lowest and it was decided that he should take an antihypertensive drug. When he took three to five bagfuls of the composition trapping radicals in organism of the present invention a day together with said antihypertensive drug, in two months, blood pressure has become stabilized of 124 to 140 as the highest and of 76 to 95 as the lowest.

(Examples 13 to 17; improving case of excessive sensitivity to cold)

(Example 13)

Female, 34 years old. She had excessive sensitivity to cold and even in summer, she used to wear socks. She also had severe tension in her shoulders. When she kept taking one bagful of the composition trapping radicals in organism of the present invention every morning, she has got warmer enough to do without socks and tension in her shoulders has been treated by rolling or by massaging.

(Example 14)

Female, 55 years old. She could not fall asleep easily since she had excessive sensitivity to cold in her left ankle. When she took two bagfuls of the composition trapping radicals in organism of the present invention before going to bed, she has got warmer from her toes right after she took the composition and she no longer needs sleeping pills.

(Example 15)

Female, 72 years old. She has been suffering from excessive sensitivity to cold for nearly 30 years and her body temperature was only 35°C on average. When she took five to six bagfuls of the composition trapping radicals in organism of the present invention a day, in three months, her body temperature has become 36.2°C, she has not caught cold, and now she can enter the air-conditioned room.

(Example 16)

Female, 68 years old. Her ankles were very cold as if she soaked them in icy water and she always carried a body warmer. When she took two bagfuls of the composition trapping radicals in organism of the present invention a day, in three months, her body has become warmer and she no longer shivers with cold even she is outside.

(Example 17)

Female, 52 years old. Due to anemia, she used to take medicine for 5 years. When she took five bagfuls of the composition trapping radicals in organism of the present invention a day, in one week, her body has become warmer and as a result of routine check up, the doctor told her she need not take medicine any longer.

(Examples 18 to 19; improving case of menstrual disorder)

(Example 18)

Female, 46 years old. She developed endometriosis and adenomyosis of uterus, and she has suffered from severe anemia due to great amount of bleeding in menstruation. When she took two bagfuls of the composition trapping radicals in organism of the present invention every after meal and when she took ten bagfuls of the composition a day in the event of severe menstruation, in two months, bleeding amount has got reduced and anemia has been alleviated.

(Example 19)

Female, 23 years old. She has been suffering from severe menstrual pain and disorder and she used to take lots of medicines. When she took five bagfuls of the composition trapping radicals in organism of the present invention a day, in two months, headache as well as menstrual disorder has been alleviated and she now greatly reduces the times of taking medicines.

(Examples 20 to 21; improving case of diabetes)

(Example 20)

Male, 55 years old. For 17 years he has been suffering from diabetic nephropathy and he has been receiving a dialysis treatment. Further, his potassium level was 6 to 6.5 and he kept taking a medicine for reducing the potassium level. Neutral fat was also high with 400 mg showing the state of hyperlipemia. When he took two bagfuls of the composition trapping radicals in organism of the present invention a day, in two months, the result of medical check up has shown that the potassium level has been lowered to a normal level of 4 and neutral fat has also been lowered to a normal level of 130mg.

(Example 21)

Female, 59 years old. She was diagnosed as diabetes by the medical check up at hospital with a blood glucose level of 301, hemoglobin of 18.7%, and with positive sugar urine. When she took three bagfuls of the composition trapping radicals in organism of the present invention a day without a dietary restriction, in two months, the result of medical check up has shown a blood glucose level of 108, hemoglobin of 7%, and with negative sugar urine. For information, she took the lightest medicine for diabetes as well.

(Examples 22 to 23; improving case of brain infarct and Parkinson's disease)

(Example 22)

Male, 82 years old. Due to aftershock of brain infarct, he had cold fingers and so he used to wear gloves. He had shaky fingers, heavy tread, and he used to shuffle forward. When he took three to five bagfuls of the composition trapping radicals in organism of the present invention, in two days, his fingers have become warmer and he could take off his gloves. He has come to walk normally with light steps. In five days, his shaky fingers have been recovered and without wearing socks, he is warm enough with bare feet.

(Example 23)

Female, 75 years old. 4 years ago, she was diagnosed as light brain infarct and she used to take a medicine for brain infarct. 3 months ago, her body movement became awkward and she was diagnosed as Parkinson' s disease. When she kept on taking three to five bagfuls of the composition trapping radicals in organism of the present invention, in sixmonths, walking has become smooth and she was able to go shopping.

(Examples 24 to 26; improving case of disease of an internal organ)

(Example 24)

Male, 54 years old. 10 years ago, he developed a hepatitis C and after that he was diagnosed as cirrhosis hepatic. His blood pressure was also high with 143 to 148 highest and with 90 to 100 lowest. When he took each bagful of the composition trapping radicals in organism of the present invention every after meal, before going to bed, and at the time of emptiness, in one month, GOT and GPT of hepatic function have been lowered to the normal range of not greater than 501 U and the blood pressure has been lowered to 122 to 133 as highest and 76 to 85 as lowest.

(Example 25)

Male, 59 years old. Several years ago, he had gallbladder polyp. However, when he took each two bagfuls of the composition trapping radicals in organism of the present invention in the morning, at noon, and in the evening, in four months, the result of the medical check up has shown he has no health problem at all.

(Example 26)

Male, 16 years old. 5 months ago, he developed Ig A nephropathy and shakiness due to a side effect by a medicine got worse.
When he took four bagfuls of the composition trapping radicals in organism of the present invention after meal and before going to bed, the next day, he found that the color of test paper for urinalysis told his health condition recovered to the normal level and up to present, for four months, he is in good shape.

(Examples 27 to 28; improving case of constipation)

(Example 27)

Female, 28 years old. She has been suffering from severe constipation. Although she tried supplement for constipation, it did not work and sometimes she had an abdominal pain caused by constipation. When she took three bagfuls of the composition trapping radicals in organism of the present invention a day, every after meal, in three months, every morning she has found constipation was prevented.

(Example 28)

Female, 79 years old. She had a difficult bowel movement with defecation every three to four days. She has been suffering from constipation accompanied with back pain. When defecation was almost impossible, she sometimes tried cleansing enema. When she took three bagfuls of the composition trapping radicals in organism of the present invention at one time, in about three minutes she blew off. After that she took three bagfuls of the composition trapping radicals in organism of the present invention three times a day, she has evacuated every day.

(Examples 29 to 35; improving case of atopic dermatitis)

(Example 29)

Female, 20 years old. She has been suffering from atopic dermatitis for 20 years and due to stress, atopy developed all over her body even to her scalp. When she took one bagful of the composition trapping radicals in organism of the present invention a day, one month after, her skin has no longer hurt when taking a shower and atopy in her sculp has disappeared. Further, swell in her face has disappeared and dusky-red skin has been recovered to dullness- free skin color.

(Example 30)

Female, 48 years old. She has been suffering from atopic dermatitis and her fingers were cracked, bleeding and she found it hard to do domestic affairs. When she took eight bagfuls of the composition trapping radicals in organism of the present invention a day, in three days, the cut was cured and bled no longer. Further, in one month, itchy feeling has disappeared as well as swell.

(Example 31)

Female, 22 years old. She has been suffering from atopy since she was in elementary school. Without applying ointment cream prescribed at the hospital even to her face, she got dry skin and could not sleep at night. When she took four bagfuls of the composition trapping radicals in organism of the present invention in total, that is, in the morning, in the evening, and at night, in seven months, itchy feeling and dryness peculiar to atopy have been alleviated and she no longer went to hospital for treatment.

(Example 32)

Male, 32 years old. He has been suffering from severe atopy since he was a child. Days and nights, he used to scratch his body and every morning he woke up to find that he was bleeding. When he took three to eight bagfuls of the composition trapping radicals in organism of the present invention a day, in three months, dryness and itchy feeling have been alleviated and he no longer scratches his body to bleed and does not tired easily.

(Example 33)

Female, 32 years old. She has been suffering from atopy since she was a child and eight years ago she stopped using steroid drugs because they are said to be bad for health. The symptom has become worse, however. When she took six to eight bagfuls of the composition trapping radicals in organism of the present invention a day, in a week to two weeks, she got a rash, but in four months, rash no longer has come out.

(Example 34)

Female, 70 years old. She has been suffering from essential pruritus which has not been improved with the hospital drugs. Especially in summer, she has been suffering from intolerable pain. When she took one bagful of the composition trapping radicals in organism of the present invention everyday, in four days, itchy feeling has almost stopped and in ten days, itchy feeling has completely stopped.

(Example 35)

Male, 71 years old. 27 years ago, he developed vulgaris infection which is a severe state of atopy caused by an injection shock. Big dandruff came out of his body. When he took two bagfuls of the composition trapping radicals in organism of the present invention a day, in three months, irritation has stopped and in six months, dandruff has stopped coming out.

(Examples 36 to 37; improving case of hay fever)

(Example 36)

Male, 60 years old. He developed a hay fever when he was 34 and since then, until 60, the symptom has been appearing every spring and autumn. Especially in autumn, he has had severe runny nose, sneeze, eye irritation, itching in the area of ears and joints. When he took four bagfuls of the composition trapping radicals in organism of the present invention a day, that is, he took each one bagful of the composition in every meal and before going to bed. Since then, he has had no symptom of a hay fever for three months.

(Example 37)

Female, 58 years old. Two years ago, she developed a hay fever and has had very uncomfortable runny nose and eye irritation. When she took three bagfuls of the composition trapping radicals in organism of the present invention, in about a week, these symptoms have disappeared.

(Examples 38 to 40; improving case of arthrorheumatism)

(Example 38)

Female, 62 years old. Due to arthrorheumatism, she had aching legs and used a stick when she walked. When she ate five to six bagfuls of the composition trapping radicals in organism of the present invention as it is or as dissolved in hot water, in eight days, pain has stopped and after that, she has relieved from pain for over 6 months.

(Example 39)

Female, 53 years old. Due to arthrorheumatism, she had stiffened fingers and found it hard to sleep at night. Also she had excessive sensitivity to cold. Right after she took one bagful of the composition trapping radicals in organism of the present invention, she felt her body warmed. And in two months, she has come to sleepwell at night. Her stiffened fingers have become straight and controllable.

(Example 40)

Female, 53 years old. Due to arthrorheumatism, she used to sleep with a mouthpiece at night. She also had pain in limbs. When she took four to five bagfuls of the composition trapping radicals in organism of the present invention a day, in a month, she has realized that her jaw and limbs no longer ached. She has come to sleep at night without a mouthpiece.

(Examples 41 to 43: improving case of bone density (bone-thinning osteoporosis and disc herniation)

(Example 41)

Female, 78 years old. She had a curved back bone and her third and fourth bones have been crushed. She had a severe pain in pelvis and found it difficult to walk. When she took three bagfuls of the composition trapping radicals in organism of the present invention a day, to be specific, one bagful thereof three times a day, in 40 days, the result of examination on bone density showed that her bone density recovered to be 102 % of the average value of female of her age.

(Example 42)

Female, 26 years old. After delivery of her second child, she was concerned about lowered bone density. Right after delivery, she took a bagful of the composition trapping radicals in organism of the present invention a day, in a month, the result of medical check-up showed her bone density was more than average despite her condition after child birth.

(Example 43)

Female, 42 years old. Diagnosed as herniation, she found it difficult to get up and difficult to work every day. When she took three bagfuls of the composition trapping radicals in organism of the present invention a day, in two months, pain in waist has completely disappeared. Wakening has become good and she has now in a good shape and therefore, enjoys working.

(Examples 44 to 46; improving case of gout and the like)

(Example 44)

Female, 75 years old. Due to gout, she had a painful big toe. However, when she took each bagful of the composition trapping radicals in organism of the present invention three times before meal, in a week, pain caused by gout has disappeared.

(Example 45)

Female, 71 years old. Due to gout, she even found it difficult to walk with painful waist and knees. When she took each bagful of the composition trapping radicals in organism of the present invention three times a day, in two weeks, painful gout has been alleviated and pain in waist and in knees has disappeared, and he can now take a walk with his dog.

(Example 46)

Male, 64 years old. Due to lumbago, he had to take a rest three times, while walking to the station for 15 minutes and he was obliged to quit his job. However, when he kept on taking 3 bagfuls of the composition trapping radicals in organism of the present invention a day, in three months, he started to work part time.

(Examples 47 to 49; improving case of autonomic ataxia)

(Example 47)

Male, 52 years old. Due to disorder of autonomic nerve, he has been suffering from indefinite complaint symptom every other day, including headache, dizziness, palpitation, breadth shortness, and breathing difficulty. When he put 2 to 4 bagfuls of the composition trapping radicals in organism of the present invention in hot water, in 4 months, initiation cycle of indefinite complaint has been reduced to about once a week, symptom has been alleviated to some extent.

(Example 48)

Female, 60 years old. Due to autonomic ataxia, she could not sleep at night. Therefore, she took medicine 4 times a day prescribed by a doctor at the hospital. When she took 5 to 6 bagfuls of the composition trapping radicals in organism of the present invention for six months consecutively, she can take medicine only once a day and she can sleep even without sleeping pills.

(Example 49)

Female, 37 years old. Due to autonomic ataxia, she has been suffered from acute headache and strong dizziness for about four years. When she took 4 to 5 bagfuls of the composition trapping radicals in organism of the present invention, in two months, headache and dizziness have been alleviated and housework and parenting has become less burdensome.

(Example 50: alleviated case of hyperventilation syndrome)

Female, 42 years old. She suffered from frequently repeated strong dizziness and attack of hyperventilation syndrome.
When she took each 2 bagfuls of the composition trapping radicals in organism of the present invention every morning and every night, in three days, she felt alleviated and in three months, even without medicine, the syndrome has been alleviated and she has suffered from no attacks any longer.

(Example 51: alleviated case of chronic disease)

Female, 30 years old. She suffered from chronic disease and took medicine for headache. However, right after she took about 3 bagfuls of the composition trapping radicals in organism of the present invention, headache has stopped and she could do without medicine for headache. In addition, tension in her shoulders has been alleviated.

(Example 52: improving case of graves disease)

Female, 35 years old. After a child birth, she developed graves disease with excess of thyroid hormone and she took medicines with strong side effects. When she took 4 bagfuls of the composition trapping radicals in organism of the present invention every morning and noon, in one month, her hormone value has stabilized to the doctor's surprise. The doctor told her that she may take only 1 tablet of medicine instead of taking 6 tablets which she used to take.

(Example 53: Improving case of cataracts)

Female, 78 years old. A doctor told her that she should take an operation for cataracts. When she took 3 bagfuls of the composition trapping radicals in organism of the present invention per day, and in ten days, she went to a doctor for diagnosis. The doctor told her that since her eyes were recovered, no operation is required.

(Example 54: Improving case of eye strain)

Female, 55 years old. She routinely suffered a severe eye strain and bloodshot eyes since her job requires to use a personal computer every day. She tentatively solved inflammation of the eye with eye drops taking as long as a week. When she took 2 bagfuls of the composition trapping radicals in organism of the present invention at one night, and in the following day, she found that inflammation disappeared.

(Example 55: Improving case of Morton's neuralgia)

Female, 46 years old. She developed hallux valgus so seriously that neuralgia of particularly a light leg caused a serious headache. An orthopedist diagnosed as Morton's neuralgia. When she took 3 bagfuls of the composition trapping radicals in organism of the present invention per day, in six days, she was freed from severe pain and the operation is no longer required.

(Experiments for confirming effects by animal monitors)

In order to confirm the improving effects of the composition trapping radicals in organism of the present invention on several symptoms of pet animals, experiments for confirming effects by pet monitors were also conducted. For monitoring, as in the human case, basically each 3 g of bagged composition trapping radicals in organism of the present invention was fed to monitors by oral intake.

(Examples 56 to 77: Effect on dogs)

(Example 56)

Hybrid with Shiba-dog, male, 13 years old. The dog suddenly developed brain infarction. The eyes began to rotate and the neck was tilted in 45°. The dog kept lying and it seemed painful. At first, a bagful of the composition trapping radicals in organism of the present invention was fed to the dog mixed with saliva of a keeper every day by putting the composition on the dog's nose, it ate up in a second. Hereinafter, likewise, when a bagful of the composition trapping radicals in organism of the present invention was fed to the dog each day and in three days, the dog began to eat dog food gradually and in two weeks, the neck has become straight and the dog has recovered its appetite and now it can take a walk.

(Example 57)

Hybrid, 8 years old. The dog suffered from cardiac disease and everyday a keeper administered hospital drugs. When a bagful of the composition trapping radicals in organism of the present invention was fed to the dog every day, and in three weeks, measurement of an electrocardiogram has shown that its heart has been recovered.

(Example 58)

Shih Tzu, male, 13 years old. Due to heart valve disease, it began to develop cough. With the prescription of a veterinarian, hydragogue and vasolidating drugs were administered to the dog, and although it stopped coughing, a keeper was concerned about the side effects. As a result that each bagful of the composition trapping radicals in organism of the present invention was fed to the dog every morning and every night, it keeps fine although cardiac sound is not good.

(Example 59)

Shih Tzu, female, 7 years old. Due to stress, it developed gastroenteritis and the dog vomited and experienced diarrhea.
When the dog was ill, one bagful of the composition trapping radicals in organism of the present invention was fed to the dog in two batches for a couple of days, the dog has recovered appetite soon and begun to eat. Therefore, it does not have to go to hospital.

(Example 60)

Labrador, 11 months old. Due to stress and eating too much of snacks, its stomach became ill. When one bagful of the composition trapping radicals in organism of the present invention was fed to the dog every day, the dog no longer complains about itch in three weeks.

(Example 61)

Middle-sized Chindo dog, female, 3 years old. All the year round, the dog was suffering from fur loss. And since it ate weed when it took a walk, it developed an allergy with fallen fur around its tail.
When water washing out ten to fifteen bags of the composition trapping radicals in organism of the present invention taken by a keeper was continuously fed to the dog once in a couple of days, its fur has begun to grow and in six months, its fur has become bushy.

(Example 62)

Shih Tzu, male, 16 years old. It developed an allergy peculiar to Shih Tzu dog with pimples all over its body. When its fur was removed for treating boils, the dog went almost bald. Since the dog is old, it developed cataracts and cardiac hypertrophy. It sometimes coughed and had no energy. When each bagful of the composition trapping radicals in organism of the present invention was fed to the dog every morning and every night, even when it coughed, in seven days the cough has stopped and now, six months from the intake of the composition trapping radicals in organism of the present invention, the dog has regained its bushy fur and it is now jumping around when it takes a walk which it used to hate.

(Example 63)

Hybrid, male, 5 years old. The cause was not certain, but the dog had no vigor and no appetite for four days. When one and a half bagful of the composition trapping radicals in organism of the present invention was fed to the dog by offering the dog in a small portion from hand, it has restored its vigor, running cheerfully and even it has got horny.

(Example 64)

Maltese, male, 18 years old. At the age of 10, the dog was liable to faint because of anemia. At the age of 13, breathing was difficult for the dog because of tracheostenosis. When the dog had bad condition, a bagful of the composition trapping radicals in organism of the present invention which a keeper bit and rounded was fed to the dog three times a day. Usually, half bagful thereof was fed to the dog. Anemia has become improved in about two months and the dog is now running vigorously despite its age, although sometimes tracheostenosis attacks it.

(Example 65)

Shiba-dog, 15 years old. The dog sometimes was coughing maybe because it did not have heartworm vaccination. When a bagful of the composition trapping radicals in organism of the present invention was fed to the dog at a time of symptom, it has stopped coughing in a couple of days.

(Example 66)

Maltese, female, 14 years old. Three months ago, the dog developed asthma and it has been coughing with strangely wheezing breath and despite the treatment at the hospital, the coughing did not stop. When half bagful of the composition trapping radicals in organism of the present invention was fed to the dog in a squashy state kneaded with tea, in one or two minutes, strange coughing has stopped. Then half bagful thereof was subsequently fed to a dog, and its condition is now stable.

(Example 67)

Maltese, female, 9 years old. Every morning the dog's nose did not stop running and the dog has no appetite, either. When a bagful of the composition trapping radicals in organism of the present invention was fed to the dog from hand, the running nose completely stopped in three hours. The dog also has restored its appetite.

(Example 68)

Pomeranian, female, 6 years old. Since two years ago, the surroundings of its eyes were sticky by the mucus from the eyes, and with aging, pigmented spot came to appear in its belly. When a half bagful of the composition trapping radicals in organism of the present invention was fed to the dog per day, the mucus has been recovered unawares and pigmented spot has disappeared and the dog has restored a white belly.

(Example 69)

Shih Tzu, male, 10 years old. Although it suffered from cataracts, when a half bagful of the composition trapping radicals in organism of the present invention was fed to the dog everyday, in four months, white turbidity in eyes has disappeared.

(Example 70)

Miniature poodle, female, 15 years old. The dog developed cataracts and had a boil of 1cm in height. Half a bagful of the composition trapping radicals in organism of the present invention was fed to the dog twice a day and in two months, the cataracts have seen 70% recovery and the boil has also been defeated and seen recovery. The dog's menstruation has restarted and it is now very agile like young dogs.

(Example 71)

Hybrid middle-sized dog, female, 14 years old. For 3 weeks, the dog was hospitalized for all extraction operations of uterus cancer and breast cancer. One week before it left hospital, a bagful of the composition trapping radicals in organism of the present invention was fed to the dog and after its discharge, two to three bagfuls thereof were fed to the dog per day and now, one bagful thereof is fed to it every other day. The dog has restored its vigor completely.

(Example 72)

Shiba-dog, male, 12 years old. 6 months ago, the dog underwent orchidotomy for treating cancer, and after the operation it couldn't move one of its legs because of numbness and the dog whined in a tragic voice since excretion became difficult against its will. For the first one month, seven bagfuls of the composition trapping radicals in organism of the present invention were fed to the dog per day, and then from the second month, five bagfuls thereof, and the third month, three bagfuls thereof, and now, one bagful thereof was fed to the dog dissolved with lukewarm water. After passage of two months, the leg has stopped quivering, and the dog has come to stand firm. After passage of three months, the dog has come to urinate normally. And now, its fur has become shiny and it has been recovered and it cannot be considered as old as 12 years.

(Example 73)

Golden retriever, 6 years old. One week after the dog received anti-cancer drugs for lymphoma, it lost its appetite and vigor by the side effects. Four bagfuls of the composition trapping radicals in organism of the present invention were fed to the dog per day, and it has rapidly got better. The dog seems younger with shiny furs than it used to be without any side effects.

(Example 74)

Hybrid, male, 14 years old. Though the dog used to discharge urine while walking in the morning and in the evening, suddenly, it suffered from incontinence regardless of the time of the day. Two days after the dog became incontinence, two bagfuls of the composition trapping radicals in organism of the present invention were fed to the dog both in the morning and in the evening and a bagful of the composition trapping radicals in organism of the present invention was fed to the dog at night kneaded with tea. Ten days after, incontinence stopped.

(Example 75)

Shiba-dog, female, 14 years old. From 2 years before, the dog had blood in the stool. A bagful of the composition trapping radicals in organism of the present invention was fed to the dog with a meal every day and the dog had no longer had blood in the stool in less than 1 week and it has recovered so well that it now runs about sprinting.

(Example 76)

Spitz, female, 13 years old or older. It became so weak that it was almost dead, tottering and spitting blood. The composition trapping radicals in organism of the present invention was sprinkled on a food in the morning and in the evening, the dog has become rapidly better recovering from the situation where the veterinarian almost gave up.

(Example 77)

Beagle, female, 1 year old. Since it was born as a premature baby, it was weak. When it had a high fever of 40 degrees centigrade, it didn' t eat anything and barely drank little water. A composition trapping radicals in organism of the present invention was mixed with food and was fed to the dog. It has gotten well even when it sent to the hospital.

(Examples 78 to 79: Effects on cats)

(Example 78)

Cat, female, 3 to 4 years old. The cat bred six kittens and these kittens were separated from a mother cat when they were 1 month old. The mother cat would not sleep, would not eat almost anything and on a third day from the separation, it spit blood as many as three times and became limp. 1/3 bagful of composition trapping radicals in organism of the present invention was mixed with a canned pet food and fed to the cat three times a day. It started to eat without resistance and hereafter, it no longer has vomited and has restored physical power.

(Example 79)

Persian cat, male, 10 years old. The cat suffered from a chronic festering ear wax which accompanied strongly foul odor and bloody pus. Despite cleansing and dosing at a hospital, it did not improve. 1/2 or 1/3 bagful of composition trapping radicals in organism of the present invention was sprinkled on a canned pet food and fed to the cat. In 10 days, strongly foul odor has weakened and in 6 months, the cat has had almost no odor and pus has decreased, too.

(Example 80: effect on a ferret (note that a ferret is a small animal of a weasel family)

Female, 3 years old. Its genitals swelled up and all the fur came off. A veterinarian diagnosed that sterilization did not go well and hormone balance was not orderly. Despite dosing for a month, it was not improved. However, as a result of dosing half a bagful of the masticated composition trapping radicals in organism everyday, in two weeks, swelling of the genitals was reduced and fuzzy hairs grew all of the body. In four months, its fur has become soft, white, and shiny.

Industrial applicability

The composition trapping radicals in organism has industrial applicability as health supplement for preventing and improving symptoms caused by blood flow obstruction including hypertension and excessive sensitivity to cold and other many symptoms as mentioned above.

The composition trapping radicals in organism is also available for health drink or food like candies or gums using its extract or essence. Further, the composition is also available for raw materials for cosmetics, soaps, shampoo, rinse, hair care products, hair dyes, make-up products, hair growth tonic, or the like.

**Claims**

1. A composition trapping radicals in an organism, said composition having been prepared from soy bean, wheat germ, adlay, husked rice germ, and rice bran, by a process which comprises roasting said materials independently at a temperature of 50 to 150°C, then steaming them independently, then fermenting them independently by adding at least one species of edible microbe selected from microbes belonging to Aspergilius oryzae to each of said materials, and after fermentation, adding fine powders of at least green tea powders, sesame, rosemary, acerola, yuzu, carrot, Gymnema Sylvestre and green barley to each of said materials, then mixing, further followed by making the mixture an ultra fine (30 to 50μm) powder.

2. A composition trapping radicals in organism as claimed in claim 1, wherein said composition comprises 25 to 40 wt. % soy bean, 25 to 35 wt. % wheat germ, 3 to 10 wt. % adlay, 3 to 20 wt. % husked rice germ, 5 to 20 wt. % rice bran, 15 to 20 wt. % green tea powders, 1 to 10 wt. % sesame powders, and 0.05 to 5 wt. % other ingredients with respect to the total amount.

3. A composition as claimed in wither claim 1 or claim 2 wherein the composition is for trapping hydroxyl radicals in blood or cells.

4. An essence of a composition for trapping radicals in an organism extracted from a composition for trapping radicals in an organism as claimed in any one of claims 1 to 3.

5. A process for preparing a composition for trapping radicals in an organism comprising
   The first step of roasting soy bean, wheat germ, adlay, husked rice germ, and rice bran, at a temperature of 50 to 150°C independently, then steaming them independently;
   the second step of fermenting each of said materials independently by adding at least one species of edible microbes selected from microbes belonging to Aspergilius oryzae to each of saidmaterials, further followed by applying cut back operation at least one to each of the fermenting materials during the fermentation process;
   the third step of adding fine powders of at least green tea, sesame, rosemary, acerola, yuzu, carrot, Gymnema Sylvestre and green barley to each of said materials and mixing; and
   the fourth step of grinding the mixture obtained in said third step into an ultra fine (30 to 50μm) powder.

6. A composition as claimed in any one of claims 1 to 3 or an essence as claimed in claim 4 for use as a medicament.

7. A composition or an essence as claimed in claim 6 for use in the treatment of a condition mediated by free radicals.

8. A composition or an essence as claimed in claim 6 for use in a human in the treatment or prophylaxis of a condition selected from the group consisting of hypertension, excessive sensitivity to cold, menstrual disorder, anaemia, diabetes, brain infarction, Parkinson's disease, hepatic cirrhosis, gall bladder polyps, IgA nephropathy, constipation, atopic dermatitis, essential pruritus, hay fever, articular rheumatism, osteroporosis, discherniation, gout, lumbago, autonomic ataxia, graves disease, cataract, eye strain, and Morton's neuralgia, or for use in a non-human mammal in the treatment or prophylaxis of a condition selected from the group consisting of brain infarction, cardiac disease, gastroenteritis, fur loss, allergy, cataract, cardiac hypertrophy, loss of appetite, anaemia, persistent cough, asthma, rhinitis, and incontinence.

9. Use of a composition as claimed in any one of claims 1 to 3 or an essence as claimed in claim 4 for the manufacture of a medicament for the treatment or prophylaxis in a human of a condition selected from the group consisting of hypertension, excessive sensitivity to cold, menstrual disorder, anaemia, diabetes, brain infarction, Parkinson's disease, hepatic cirrhosis, gall bladder polyps, IgA nephropathy, constipation, atopic dermatitis, essential pruritus, hay fever, articular rheumatism, osteroporosis, disc herniation, gout, lumbago, autonomic ataxia, graves disease, cataract, eye strain, and Morton's neuralgia, or the treatment or prophylaxis in a non-human mammal of a condition selected from the group consisting of brain infarction, cardiac disease, gastroenteritis, fur loss, allergy, cataract, cardiac hypertrophy, loss of appetite, anaemia, persistent cough, asthma, rhinitis, and incontinence.

【FIG.1】

【FIG.2】

Fig.2.  Effect of (+)-catechin and SMeEx on high blood glucose level induced by alloxan in mice. Symbols indicate as follows:

◆,NT ;  ▨,control(cont);  ■ ,(+)-catechin(CA)100mg/kg i.p.;   ▲,SMeEx 200mg/kg i.p.

Each point represents the mean  ±S.E. of 8-15 experiments.

*Significantly different from the corresponding control at p＜0.05, and **: p＜0.01.

【FIG.3】

## Change in blood glucose level

Fig.3. Effect of (+)-catechin and SMeEx on high blood glucose level induced by alloxan in mice. Symbols indicate as follows:

,control(cont); ▲,(+)-catechin(CA) 100mg/kg i.p.; ,SMeEx 100mg/kg i.p. Each point represents the mean ±S.E. of 15 or 16 experiments.

*Significantly different from the corresponding control at $p < 0.05$.

【FIG.4】

Lipid peroxide levels of erythrocyte membranes on 12th day

Fig.4: Boxplots in lipid peroxide levels of erythrocyte memmbranes from alloxan diabetes mice. ∗: Significantly different from the corresponding control at p＜0.05 and ∗∗: P＜0.01.

【FIG.5】

Change in body weight

**EP 1 588 711 A1**

**European Patent Office**

**EUROPEAN SEARCH REPORT**

Application Number

EP 05 25 2505

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DATABASE WPI<br>Section Ch, Week 199710<br>Derwent Publications Ltd., London, GB;<br>Class B04, AN 1997-103658<br>XP002337713<br>& JP 08 337536 A (ASAHI BREWERIES LTD)<br>24 December 1996 (1996-12-24)<br>* abstract * | 1-9 | A61K35/78<br>A61K45/06<br>A61P39/06<br>A61P9/12 |
| X | DATABASE WPI<br>Section Ch, Week 199538<br>Derwent Publications Ltd., London, GB;<br>Class B04, AN 1995-290315<br>XP002337714<br>& JP 07 188044 A (ASAHI BREWERIES LTD)<br>25 July 1995 (1995-07-25)<br>* abstract *<br>& JP 07 188044 A (ASAHI BREWERIES LTD)<br>25 July 1995 (1995-07-25) | 1-9 | |
| X | DATABASE WPI<br>Section Ch, Week 200338<br>Derwent Publications Ltd., London, GB;<br>Class B04, AN 2003-397308<br>XP002337715<br>& JP 2002 326945 A (AOA JAPAN CO LTD)<br>15 November 2002 (2002-11-15)<br>* abstract *<br>& JP 2002 326945 A (AOA JAPAN:KK)<br>15 November 2002 (2002-11-15) | 1-9 | TECHNICAL FIELDS SEARCHED (Int.Cl.7)<br><br>A61K<br>A61P |

-/--

The present search report has been drawn up for all claims

| Place of search | Date of completion of the search | Examiner |
|---|---|---|
| Munich | 25 July 2005 | Escolar Blasco, P |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

30

**European Patent Office**

## EUROPEAN SEARCH REPORT

Application Number

EP 05 25 2505

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DATABASE WPI<br>Section Ch, Week 199226<br>Derwent Publications Ltd., London, GB;<br>Class B04, AN 1992-211992<br>XP002337716<br>& JP 04 139132 A (ASAHI BREWERIES LTD)<br>13 May 1992 (1992-05-13)<br>* abstract *<br>----- | 1-9 | |
| X | DATABASE WPI<br>Section Ch, Week 199213<br>Derwent Publications Ltd., London, GB;<br>Class B04, AN 1992-099832<br>XP002337717<br>& JP 04 041436 A (SODEX KK)<br>12 February 1992 (1992-02-12)<br>* abstract *<br>----- | 1-9 | |

TECHNICAL FIELDS SEARCHED (Int.Cl.7)

The present search report has been drawn up for all claims

| Place of search | Date of completion of the search | Examiner |
|---|---|---|
| Munich | 25 July 2005 | Escolar Blasco, P |

EPO FORM 1503 03.82 (P04C01)

**ANNEX TO THE EUROPEAN SEARCH REPORT
ON EUROPEAN PATENT APPLICATION NO.**

EP 05 25 2505

This annex lists the patent family members relating to the patent documents cited in the above-mentioned European search report.
The members are as contained in the European Patent Office EDP file on
The European Patent Office is in no way liable for these particulars which are merely given for the purpose of information.

25-07-2005

| Patent document cited in search report | | | Publication date | Patent family member(s) | | Publication date |
|---|---|---|---|---|---|---|
| JP 8337536 | A | | 24-12-1996 | NONE | | |
| JP 7188044 | A | | 25-07-1995 | NONE | | |
| JP 2002326945 | A | | 15-11-2002 | NONE | | |
| JP 4139132 | A | | 13-05-1992 | JP | 2080198 C | 09-08-1996 |
| | | | | JP | 7119176 B | 20-12-1995 |
| JP 4041436 | A | | 12-02-1992 | NONE | | |

EPO FORM P0459

For more details about this annex : see Official Journal of the European Patent Office, No. 12/82